(19) Europäisches Patentamt / European Patent Office / Office européen des brevets

(11) **EP 3 157 954 B1**

(12) **FASCICULE DE BREVET EUROPEEN**

(45) Date de publication et mention de la délivrance du brevet:
**25.12.2019 Bulletin 2019/52**

(21) Numéro de dépôt: **15738731.7**

(22) Date de dépôt: **18.06.2015**

(51) Int Cl.:
*C07K 16/24* (2006.01)     *A61P 37/06* (2006.01)
*A61K 39/395* (2006.01)

(86) Numéro de dépôt international:
**PCT/FR2015/051616**

(87) Numéro de publication internationale:
**WO 2015/193622 (23.12.2015 Gazette 2015/51)**

(54) **COMPOSITION ORALE D'ANTICORPS ANTI-TNFALPHA**

ORALE ZUSAMMENSETZUNG VON ANTI-TNF-ALPHA-ANTIKÖRPERN

ORAL COMPOSITION OF ANTI-TNF ALPHA ANTIBODIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.06.2014 FR 1455582**

(43) Date de publication de la demande:
**26.04.2017 Bulletin 2017/17**

(73) Titulaire: **Laboratoire Français du Fractionnement et des Biotechnologies
91940 Les Ulis (FR)**

(72) Inventeur: **CHTOUROU, Abdessatar Sami
F-78990 Elancourt (FR)**

(74) Mandataire: **Cabinet Becker et Associés
25, rue Louis le Grand
75002 Paris (FR)**

(56) Documents cités:
**WO-A1-2009/046168     WO-A1-2011/047328
WO-A2-2004/048517     WO-A2-2014/125374**

- BHOL K C ET AL: "AVX-470: A novel oral Anti-TNF antibody with therapeutic potential in inflammatory bowel disease", INFLAMMATORY BOWEL DISEASES, WILLAMS AND WILKINS, RAVEN PRESS, HAGERSTOWN, MD; US, vol. 19, no. 11, 1 octobre 2013 (2013-10-01), pages 2273-2281, XP009176810, ISSN: 1078-0998
- Yann Echelard: "Production in the Milk of Transgenic Animals A Validated, Cost-Effective Approach for the Manufacturing of Complex Recombinant Protein", Eurobion 2009, 1 janvier 2009 (2009-01-01), pages 1-34, XP055169403, Extrait de l'Internet: URL:http://www.eurobio-event.com/DocBD/speaker/pdf/83.pdf [extrait le 2015-02-12]
- WALTER L. HURLEY ET AL: "Perspectives on Immunoglobulins in Colostrum and Milk", NUTRIENTS, vol. 3, no. 12, 14 avril 2011 (2011-04-14), pages 442-474, XP055169533, DOI: 10.3390/nu3040442

Remarques:
Le dossier contient des informations techniques présentées postérieurement au dépôt de la demande et ne figurant pas dans le présent fascicule.

**Description**

Le présent document concerne l'administration par voie orale d'un anticorps anti-facteur de nécrose tumorale alpha (TNFα). Les applications thérapeutiques visées comprennent notamment le traitement de maladies inflammatoires de l'intestin, telles que la maladie de Crohn.

**Arrière-plan technologique de l'invention** :

Le TNFα est une cytokine pro-inflammatoire qui est sécrétée par et interagit avec les cellules du système immunitaire. Le TNFα a été montré comme étant impliqué dans de nombreuses maladies humaines, notamment des maladies inflammatoires chroniques comme l'arthrite rhumatoïde, la maladie de Crohn, la colique ulcéreuse, ou encore la sclérose en plaques.

Plusieurs anticorps anti-TNFa sont actuellement en développement. Deux anticorps sont déjà commercialisés : l'infliximab (Remicade®), et l'adalimumab (Humira®), sous formes injectables par voie sous-cutanée ou intraveineuse.

Plusieurs études ont néanmoins rapporté que la thérapie par anticorps anti-TNFa, notamment par voie systémique, pouvait présenter des effets secondaires indésirables, notamment la survenue d'infections bactériennes telles que la tuberculose (Jarequi-Amezaga et al., Journal of Crohn's and colitis, 2013, 7(3) :208-212), ou d'infections par la *Listeria* (Abreu et al., Journal of Crohn's and colitis, 2013, 7(2) :175-182) ou par *Candida* (Huang et al., Journal of pediatric gastroentology and nutrition, 2013, 56(4) :e23-6).

Des formulations d'anticorps polyclonaux anti-TNFa pour une administration par voie orale sont en développement. Mais il existe encore un besoin d'améliorer l'efficacité et/ou l'innocuité des anticorps anti-TNFa administrés par voie orale.

**Résumé de l'invention**

Les inventeurs proposent maintenant une composition pharmaceutique pour administration orale dans le traitement d'une maladie inflammatoire de l'intestin comprenant un anticorps anti-facteur de nécrose tumorale alpha (TNFα), de préférence un anticorps monoclonal produit dans le lait d'un animal non-humain transgénique, et qui est combiné de manière avantageuse avec de l'acide caprylique.

La composition est sous une forme adaptée à une libération de l'anticorps ciblée au niveau de l'intestin.

Dans un mode de réalisation préféré, l'anticorps anti-TNFa est l'adalimumab ou possède la séquence protéique de l'adalimumab.

La composition est utile notamment dans le traitement d'une maladie inflammatoire, de préférence une maladie inflammatoire de l'intestin, de préférence encore la maladie de Crohn.

**Description détaillée de l'invention**

*Définitions :*

Le terme " traitement " ou " traiter " désigne une amélioration, la prophylaxie, ou l'inversion d'une maladie ou d'un trouble, ou au moins d'un symptôme pouvant être discerné de celui-ci, ou une amélioration, la prophylaxie, ou l'inversion d'au moins un paramètre physique mesurable associé à la maladie ou au trouble étant traité, qui n'est pas nécessairement discernable chez ou par le sujet traité. Le terme "traitement " ou " traiter " comprend encore l'inhibition ou le ralentissement de la progression d'une maladie ou un trouble, physiquement, par exemple, la stabilisation d'un symptôme discernable, physiologiquement, par exemple, la stabilisation d'un paramètre physique, ou les deux.

Au sens de la présente invention, par « patient » on entend tout mammifère, et plus particulièrement les êtres humains, hommes ou femmes, quel que soit l'âge.

Le terme « maladie inflammatoire » inclut toute maladie à composante inflammatoire, de préférence les maladies inflammatoires de l'intestin, notamment les maladies inflammatoires chroniques de l'intestin, telles que la maladie de Crohn et la colite ulcéreuse. Sont également comprises les arthrites juvéniles idiopathiques, les polyarthrites rhumatoïdes, le psoriasis, les rhumatismes psoriasiques, et les spondylarthrites ankylosantes.

Les termes «combinaison» et « co-administration » se rapportent à l'administration de l'anticorps anti-TNFa et de l'acide caprylique sous la forme d'une formulation unique ou comme deux formulations distinctes. L'administration peut être simultanée ou séquentielle, dans n'importe quel ordre. Les deux agents peuvent être administrés simultanément ou successivement.

*Anticorps anti-TNFα:*

Par «anticorps anti-TNFα», on entend tout anticorps se liant spécifiquement au TNFα humain. De préférence, l'anticorps se dissocie du TNFα humain selon les constantes suivantes: Kd inférieur à $1 \times 10^{-8}$ M (de préférence inférieur à $1 \times 10^{-9}$ M, de préférence inférieur à $1 \times 10^{-10}$ M, de préférence inférieur à $1 \times 10^{-11}$ M) et Koff de $1 \times 10^{-3}$ $s^{-1}$ ou moins, toutes deux déterminées par un test de résonance des plasmons de surface (« surface plasmon resonance »). De préférence l'anticorps est neutralisant, en particulier il neutralise la fonction biologique du TNFα en bloquant son interaction avec les récepteurs du TNF p55 et p75 situés à la surface cellulaire. La capacité de neutralisation de l'anticorps peut être testée par un test standard, par exemple l'anticorps neutralise la cytotoxicité du TNFα humain dans un test standard in vitro L929, avec une IC50 de $1 \times 10^{-7}$M ou moins, de préférence inférieur à $1 \times 10^{-8}$M, $1 \times 10^{-9}$M, ou $1 \times 10^{-10}$M.

De préférence il s'agit d'un anticorps monoclonal.

L'anticorps peut être un anticorps d'un mammifère tel qu'une souris, ou peut-être de préférence humanisé, ou encore entièrement humain.

Dans un mode de réalisation préféré, l'anticorps anti-TNFα est un anticorps monoclonal humain.

L'anticorps anti-TNFa préférentiellement utilisé est l'adalimumab ou possède la séquence protéique de l'adalimumab, à savoir comprend la séquence de chaîne légère SEQ ID NO : 1, et la séquence de chaîne lourde SEQ ID NO :2.

## SEQ ID NO: 1

1 DIQMTQSPSS LSASVGDRVT ITCRASQGIR NYLAWYQQKP GKAPKLLIYA ASTLQSGVPS

61 RFSGSGSGTD FTLTISSLQP EDVATYYCQR YNRAPYTFGQ GTKVEIKRTV AAPSVFIFPP

121 SDEQLKSGTA SVVCLLNNFY PREAKVQWKV DNALQSGNSQ ESVTEQDSKD STYSLSSTLT

181 LSKADYEKHK VYACEVTHQG LSSPVTKSFN RGEC

## SEQ ID NO: 2

1 EVQLVESGGG LVQPGRSLRL SCAASGFTFD DYAMHWVRQA PGKGLEWVSA ITWNSGHIDY

61 ADSVEGRFTI SRDNAKNSLY LQMNSLRAED TAVYYCAKVS YLSTASSLDY WGQGTLVTVS

121 SASTKGPSVF PLAPSSKSTS GGTAALGCLV KDYFPEPVTV SWNSGALTSG VHTFPAVLQS

181 SGLYSLSSVV TVPSSSLGTQ TYICNVNHKP SNTKVDKKVE PKSC

L'adalimumab est une immunoglobuline G (IgG) composée de deux chaînes légères kappa et de deux chaînes lourdes IgG1.

L'anticorps peut être également l'infliximab ou le golimumab par exemple.

L'anticorps utilisé dans l'invention peut être produit par toute technique connue de l'homme du métier, de préférence il s'agit d'un anticorps recombinant.

Dans un mode de réalisation particulier, l'anticorps peut ainsi être produit par recombinaison dans une cellule hôte, transformée avec un ou des vecteur(s) qui permettent l'expression et/ou la sécrétion des séquences nucléotidiques codant pour la chaîne lourde et/ou la chaîne légère de l'anticorps. Le vecteur comporte généralement un promoteur, des signaux d'initiation et de terminaison de la traduction, ainsi que des régions appropriées de régulation de la transcription. Il est maintenu de façon stable dans la cellule hôte et peut éventuellement posséder des signaux particuliers qui spécifient la sécrétion de la protéine traduite. Ces différents éléments sont choisis et optimisés par l'homme du métier en fonction de l'hôte cellulaire utilisé. De tels vecteurs sont préparés par des méthodes couramment utilisées par l'homme du métier, et les clones résultant peuvent être introduits dans un hôte approprié par des méthodes standard, telles que la lipofection, l'électroporation, l'utilisation d'agents polycationiques, le choc thermique, ou des méthodes chimiques. L'hôte cellulaire peut être choisi parmi des systèmes procaryotes ou eucaryotes, par exemple les cellules bactériennes mais également les cellules de levure ou les cellules animales, en particulier les cellules de mammifères. Les cellules de mammifère préférées pour la production de l'anticorps monoclonal sont la lignée de rat YB2/0, la lignée de hamster CHO, en particulier les lignées CHO dhfr- et CHO Lec13, PER.C6TM (Crucell), 293, K562, NS0, SP2/0, BHK ou COS. On peut également utiliser des cellules d'insectes.

Un autre mode de production est l'expression de l'anticorps recombinant dans des organismes transgéniques, par exemple dans les plantes (Ayala M, Gavilondo J, Rodríguez M, Fuentes A, Enríquez G, Pérez L, Cremata J, Pujol M. Production of plantibodies in Nicotiana plants. Methods Mol Biol. 2009;483:103-34.) ou surtout dans le lait d'animaux transgéniques tels que la lapine, la chèvre ou le porc (Pollock, D.P., J.P. Kutzko, E. Birck-Wilson, J.L. Williams, Y. Echelard and H.M. Meade. (1999). Transgenic milk as a method for the production of recombinant antibodies. Journal of Immunological Methods. 231: 147-157).

Selon un mode de réalisation préféré, l'anticorps est produit dans le lait de mammifères transgéniques non humains, modifiés génétiquement pour produire cette glycoprotéine. Le mammifère peut être par exemple une chèvre, brebis, les femelles du bison, buffle, chameau, lama, souris, rat, ou encore une vache, truie, lapine, ou jument.

De préférence, il s'agit du lait de chèvre transgénique.

La sécrétion de l'anticorps par les glandes mammaires, permettant sa présence dans le lait du mammifère transgénique, implique le contrôle de l'expression de l'anticorps de manière tissu-dépendante. De telles méthodes de contrôle sont bien connues de l'homme du métier. Le contrôle de l'expression est effectué grâce à des séquences permettant l'expression de la glycoprotéine dans un tissu particulier de l'animal. Il s'agit notamment des séquences promotrices de type « WAP », « β-caséine », « β-lactoglobuline » et éventuellement des séquences de type peptide signal. De préférence, on produit l'anticorps dans les glandes mammaires d'une chèvre transgénique, en utilisant un vecteur d'expression comprenant la séquence des deux chaînes, sous le contrôle d'un promoteur 5' β-caséine. Un procédé d'extraction de protéines d'intérêt à partir du lait d'animaux transgénique est décrit dans le brevet EP 0 264 166.

De manière avantageuse, il est produit plus de 4 grammes d'anticorps par litre de lait, de manière avantageuse plus de 5, 10, 15, 20, 25, 30, 35 grammes par litre, de manière avantageuse jusqu'à 70 grammes par litre.

De manière avantageuse, l'anticorps produit par transgénèse animale, en particulier dans les glandes mammaires d'une chèvre transgénique, est sous la forme d'une population d'anticorps anti-TNFα, qui présentent une glycosylation avec un taux de galactosylation élevé, par exemple supérieur à 60%, de préférence supérieur 70%, de préférence encore d'au moins 80%.

Selon encore un autre aspect particulier, le taux de fucosylation de l'ensemble des anticorps de la population est d'au moins 50%, et notamment d'au moins 60%.

Selon un autre aspect particulier, la population comprend des anticorps qui comprennent des N-glycannes mono-galactosylés.

Selon un autre aspect particulier, la population comprend des anticorps qui comprennent des N-glycannes bi-galactosylés.

Selon un autre aspect particulier, le rapport du taux de galactosylation des anticorps de la population et du taux de fucosylation des anticorps de la population est compris de 1,0 à 1,4.

Selon un autre aspect particulier, au moins 35% des anticorps dans la population comprend des N-glycannes bi-galactosylés et au moins 25% des anticorps dans la population comprend des N-glycannes mono-galactosylés.

Selon un autre aspect particulier, le taux de sialylation des anticorps est d'au moins 50%, de préférence d'au moins 70%, ou encore d'au moins 90%.

Selon encore un autre aspect particulier, les anticorps sont totalement sialylés.

La biosynthèse des N-glycannes n'est pas régulée par une codification, comme cela est le cas avec les protéines, mais est principalement dépendante de l'expression et de l'activité des glycosyltransférases spécifiques dans une cellule. Ainsi, une glycoprotéine, tel le fragment Fc d'un anticorps, existe normalement comme une population hétérogène de glycoformes qui portent différents glycannes sur le même squelette de protéines.

Une population d'anticorps hautement galactosylés, est une population d'anticorps dans laquelle le taux de galactosylation de l'ensemble des anticorps de la population est d'au moins 50%, d'au moins 60%, d'au moins 70%, d'au moins 80%, d'au moins 90%, jusqu'à 100% de galactosylation.

Selon un mode particulier de la population d'anticorps hautement galactosylés, le taux de galactosylation de l'ensemble des anticorps de la population est d'au moins 60%.

Le taux de galactosylation peut être déterminé avec la formule suivante :

$$\frac{\sum_{i=1}^{n} (nombre\ de\ Gal)\ * \ (\%\ surface\ relative)}{\sum_{i=1}^{n} (nombre\ de\ A)\ * \ (\%\ surface\ relative)} * 100$$

dans laquelle :

- « n » représente le nombre de pics N-glycannes analysés sur un chromatogramme, par exemple d'un spectre de chromatographie en phase liquide à haute performance en phase normale (NP HPLC),
- « nombre de Gal » représente le nombre de galactoses sur l'antenne du glycanne correspondant au pic,
- « nombre de A » représente le nombre de motifs N-acétyl-glucosamine sur l'antenne de la forme glycannique correspondant au pic (à l'exclusion des deux motifs N-acétyl-glucosamine de la structure charpente commun des glycannes), et
- « % surface relative » correspond au pourcentage de l'aire sous le pic correspondant.

Le taux de galactosylation des anticorps de la population d'anticorps peut être déterminé, par exemple, en libérant les N-glycannes des anticorps, en résolvant les N-glycannes sur un chromatogramme, en identifiant le motif d'oligosaccharide du N-glycanne qui correspond à un pic spécifique, en déterminant l'intensité du pic et en appliquant les données à la formule mentionnée ci-dessus.

Des anticorps qui sont galactosylés incluent des anticorps qui ont des N-glycannes mono-galactosylés et des N-glycannes bi-galactosylés.

Selon un aspect particulier de la population d'anticorps hautement galactosylés, la population comprend des anticorps qui comprennent des N-glycannes mono-galactosylés, qui peuvent ou non être sialylés. Selon un aspect particulier de la population d'anticorps hautement galactosylés, au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 100% des N-glycannes des anticorps comprennent des N-glycannes mono-galactosylés. Selon encore un mode particulier de l'invention, dans la population d'anticorps hautement galactosylés, au moins 25% des anticorps comprennent des N-glycannes mono-galactosylés.

Selon un aspect particulier de la population d'anticorps hautement galactosylés, la population comprend des anticorps qui comprennent des N-glycannes bi-galactosylés, qui peuvent ou non être sialylés. Selon un aspect particulier de la population d'anticorps hautement galactosylés, au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 100% des N-glycannes des anticorps comprennent des N-glycannes bi-galactosylés. Selon encore un mode particulier de l'invention, dans la population d'anticorps hautement galactosylés, au moins 35% des anticorps comprennent des N-glycannes bi-galactosylés.

Selon encore un autre aspect de la population d'anticorps hautement galactosylés, la population comprend des anticorps qui comprennent des N-glycannes mono-galactosylés, qui peuvent ou non être sialylés, et des anticorps qui comprennent des N-glycannes bi-galactosylés, qui peuvent ou non être sialylés.

Selon un aspect particulier de la population d'anticorps hautement galactosylés, au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 99% des N-glycannes des anticorps comprennent des N-glycannes mono-galactosylés, et au moins 1%, au moins 5%, au moins 10%, au moins 15%, au moins 20%, au moins 25%, au moins 30%, au moins 40%, au moins 50%, au moins 60%, au moins 70%, au moins 80%, au moins 90%, jusqu'à 99% des N-glycannes des anticorps comprennent des N-glycannes bi-galactosylés. Selon un autre aspect particulier de la population d'anticorps hautement galactosylés, au moins 25% des anticorps comprennent des N-glycannes mono-galactosylés, et au moins 35% des anticorps comprennent des N-glycannes bi-galactosylés.

Dans un mode de réalisation préféré, l'anticorps anti-TNF$\alpha$, est produit par les cellules épithéliales mammaires d'un mammifère transgénique pour la production dans la glande mammaire d'anticorps anti-TNF$\alpha$, exogène et éventuellement aussi transgénique pour la production de sialyl-transférase. Les anticorps thérapeutiques produits ainsi présentent un taux de galactosylation élevé, et éventuellement des taux augmentés de liaisons terminales d'acide sialique alpha-2,6 sur leurs résidus glycanes liés au fragment Fc.

Dans certains modes de réalisation, l'anticorps présente un profil de glycosylation à teneur élevée en mannose. Tel qu'utilisé ici, un « profil de glycosylation à forte teneur en mannose » désigne un anticorps qui contient au moins un oligomannose ou une composition d'anticorps dans laquelle au moins 30% de l'anticorps contient au moins un oligomannose. Dans certains modes de réalisation au moins 30%, 40%, 50%, 60%, 70%, 80%, 90% ou plus des sucres des anticorps sont des oligomannoses non fucosylés. Dans d'autres modes de réalisation moins de 50%, 40%, 30%, 20%, 10%, 5% ou moins des sucres des anticorps contiennent du fucose. Dans un autre mode de réalisation, les anticorps ont une faible teneur en fucose et une forte teneur en oligomannose. Ainsi, dans d'autres modes de réalisation, au moins 30%, 40%, 50%, 60%, 70%, 80% ou 90% ou plus des sucres des anticorps sont de l'oligomannose et moins de 50%, 40%, 30%, 20%, 10% ou 5% des sucres des anticorps contiennent du fucose. Ainsi, dans un autre mode de réalisation au moins 30%, 40%, 50%, 60%, 70%, 80% ou 90% ou plus des sucres des anticorps sont de l'oligomannose non fucosylé et moins de 50%, 40%, 30%, 20%, 10% ou 5% des sucres des anticorps contiennent du fucose.

Selon un aspect particulier, les oligosaccharides contenant du mannose vont de Man5 à Man9, avec le numéro comme indicateur du nombre de résidus mannose. Par exemple, des oligosaccharides contenant du mannose peuvent inclure Man5, Man6, Man7, Man8 et Man9. Dans certains modes de réalisation, l'anticorps, tel que l'adalimumab produit de manière transgénique présente une haute teneur en Man6. Dans certains modes de réalisation, le sucre majeur est le Man5. Dans certains modes de réalisation, au moins 10%, 15%, ou plus des sucres sont du Man5. Avantageusement, au moins 20% des sucres sont du Man5. Dans d'autres modes de réalisation, le sucre majeur est le Man6. Dans certains modes de réalisation, au moins 10%, 15%, ou plus des sucres de l'anticorps produit de manière transgénique sont du Man6. Avantageusement, au moins 20% des sucres sont du Man6. Dans d'autres modes de réalisation, le sucre majeur est le Man7. Dans certains modes de réalisation, au moins 10%, 15% ou plus des sucres sont du Man7. Avantageusement, au moins 20% des sucres sont du Man7.

Sont particulièrement préférés les anticorps qui présentent un profil à fort taux de galactose ou mannose, comme décrits ci-dessus, en ce qu'ils présentent une forte affinité pour le récepteur Fc$\gamma$RIIIa (CD16). Par forte affinité, on désigne une affinité au moins égale à $2x10^6$ M$^{-1}$, de préférence au moins égale à $2x10^7$ M$^{-1}$, $2x10^8$ M$^{-1}$ ou $2x10^9$ M$^{-1}$, telle que déterminée par l'analyse Scatchard ou la technologie BIAcore (Label-free surface plasmon resonance based technology). Ce récepteur est trouvé sur de nombreuses cellules immunitaires, parmi lesquelles les cellules tueuses naturelles, les macrophages, les neutrophiles, et les cellules mastocytaires.

Cette affinité pour CD16 permet une amélioration des activités de cytotoxicité dépendante du complément (CDC), ou à médiation cellulaire dépendante des anticorps (ADCC) ou encore des phénomènes de phagocytose des cellules cibles, par rapport à des anticorps non hautement galactosylés ou non hautement mannosylés. Dans certains modes de réalisation, les populations d'anticorps anti-TNF$\alpha$, produites dans des cellules épithéliales de glandes mammaires sont supérieures, en termes de liaison au TNF$\alpha$ soluble, aux anticorps produits dans des cellules qui ne sont pas des cellules épithéliales de glandes mammaires. Dans certains modes de réalisation, les populations d'anticorps anti-TNF$\alpha$, produites dans des cellules de glandes mammaires sont supérieures, en termes de liaison au TNF$\alpha$ transmembranaire, aux anticorps produits dans des cellules qui ne sont pas des cellules épithéliales de glandes mammaires. Les tests pour déterminer le niveau de liaison au TNF$\alpha$ soluble ou au TNF$\alpha$ transmembranaire sont bien établis (voir par exemple Horiuchi et al., Rheumatology et al. 49 :1215).

*Combinaison avec de l'acide caprylique :*

L'anticorps anti-TNF$\alpha$, qui est de préférence un anticorps monoclonal produit dans le lait d'un animal non-humain transgénique, est administré en association avec de l'acide caprylique.

L'acide caprylique, aussi appelé acide octanoïque, est un acide gras saturé à chaîne linéaire comportant huit atomes de carbones.

L'acide caprylique peut être utilisé sous forme acide, ou sous la forme de sel de caprylate.

Tout sel pharmaceutiquement acceptable peut être envisagé, par exemple du sodium ou du potassium de caprylate.

Selon l'invention, mais sans vouloir être lié à un mécanisme d'action particulier, l'acide caprylique a pour effet de procurer une activité anti-septique (notamment anti-bactérienne, anti-fongique et anti-virale) in situ, et peut prévenir avantageusement les risques d'infections ou surinfections suite à la neutralisation du TNF$\alpha$ dans la lumière intestinale. La combinaison de l'anticorps anti-TNF et d'acide caprylique présente en outre un anti-inflammatoire amélioré. L'acide caprylique permet également d'améliorer l'efficacité de l'anticorps en protégeant celui-ci contre une dégradation trop rapide dans le tractus gastrointestinal, ce qui permet *in fine* de renforcer encore l'effet anti-inflammatoire de la composition.

L'invention a donc aussi pour objet une composition pharmaceutique pour administration orale, comprenant un anticorps anti- TNF$\alpha$ et de l'acide caprylique ou un sel de caprylate.

Aussi est dévoilé un anticorps anti-TNFa pour une utilisation dans le traitement d'une maladie inflammatoire par administration par voie orale, en combinaison avec une administration d'acide caprylique (ou un sel de caprylate) par voie orale.

L'anticorps et l'acide caprylique (ou un sel de caprylate) sont destinés à une administration séparée, simultanée ou séquentielle.

Un autre objet de l'invention est un kit comprenant, au sein d'un même emballage :

- Un premier récipient contenant une composition pharmaceutique pour administration orale, comprenant un anticorps anti-TNFa, ladite composition pharmaceutique étant adaptée à une libération de l'anticorps ciblée au niveau de l'intestin ;

- Un deuxième récipient contenant une composition pharmaceutique pour administration orale, comprenant de l'acide caprylique (ou un sel de caprylate).

L'anticorps anti-TNFa et l'acide caprylique (ou un sel de caprylate), peuvent être combinés au sein d'une même composition pharmaceutique, ou être utilisés sous formes de compositions séparées, administrables de manière simultanée ou séquentielle. En particulier ils peuvent être administrés séparément, mais concomitamment.

L'acide caprylique (ou un sel de caprylate) peut être utilisé dans l'invention sous forme de composition pharmaceutique ou de composition de type complément alimentaire.

*Formulations et applications thérapeutiques :*

Les compositions peuvent se présenter sous toutes les formes galéniques normalement utilisées pour une administration orale, notamment sous forme de comprimés, de gélules, de capsules, de poudre ou de toute forme pour préparation orale solide ou sous toute forme de préparation buvable.

La composition se présente sous une forme galénique adaptée à une libération ciblée au niveau de l'intestin. Par « intestin » on entend ici toutes les parties de l'intestin, en particulier le côlon. De telles compositions sont particulièrement utiles dans le traitement des maladies inflammatoires de l'intestin, car elles permettent une action locale au site de l'infection (notamment intestin grêle ou côlon). Elles limitent en outre le passage des anticorps dans la circulation sanguine, limitant les effets secondaires liés aux anticorps anti-TNFα.

Plusieurs stratégies existent pour préparer des médicaments administrés par voie orale, dont le ou les principes actifs ne sont libérés qu'au niveau de l'intestin, de préférence qu'au niveau du côlon.

Certaines stratégies comprennent la liaison covalente du médicament avec un support, le revêtement avec des polymères sensibles au pH. Peuvent aussi être utilisés des excipients et véhicules qui sont dégradés par les bactéries du côlon.

Dans un mode de réalisation particulier, l'anticorps anti-TNFα, peut être formulé sous des formes posologiques solides, comme des comprimés ou gélules, enrobés de polymères sensibles au pH. Le pH est de l'ordre de 1 à 3 dans l'estomac mais il augmente dans l'intestin grêle et le côlon pour atteindre des valeurs proches de 7. Les polymères les plus couramment utilisés, insolubles dans un pH acide mais solubles dans un pH neutre ou alcalin, sont des dérivés de l'acide méthacrylique, notamment les polymères Eudragit® L et S.

Dans un autre mode de réalisation, on peut utiliser des formulations qui sont revêtues avec des polymères dégradables par les micro-organismes du côlon (plus particulièrement par les enzymes bactériennes comme des azoréductases et glycosidases), par exemple des polymères azoïques ayant un degré élevé d'hydrophilie.

Des gels et hydrogels peuvent également être utilisés, notamment des hydrogels à base de polysaccharides.

Lorsque l'anticorps anti-TNFα, est associé à de l'acide caprylique (ou sel de caprylate), la forme posologique comprenant l'anticorps anti-TNFα, peut être différente de la forme posologique comprenant l'acide caprylique éventuellement associé.

De préférence l'anticorps anti-TNFα, est administré sous la forme d'un comprimé ou d'une gélule. De préférence, l'acide caprylique est administré sous forme de gélule.

Les compositions de l'invention peuvent comprendre ou être associées à d'autres agents thérapeutiques, mais cela de manière non préférée.

Les compositions selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées éventuellement au moyen de formes galéniques ou de dispositifs assurant une libération prolongée et/ou retardée. Pour ce type de formulation, on utilise avantageusement un agent tel que la cellulose, des carbonates ou des amidons.

Les doses administrées peuvent variées, entre autres, selon le poids et l'âge du patient, et la sévérité de la maladie, appréciée par l'homme du métier.

Dans un mode de réalisation préféré, le dosage de l'anticorps est dans la gamme d'environ 0.02mg/kg à environ 100mg/kg de poids corporel, soit d'environ 1mg à environ 8g par jour pour une personne de 55 à 80 kg.

Dans un mode de réalisation encore préféré, le dosage de l'anticorps est dans la gamme d'environ 0.16 mg/kg à environ 32 mg/kg de poids corporel, soit d'environ 8.75 mg à environ 2,5 g par jour. Dans un mode de réalisation encore préféré, le dosage de l'anticorps est dans la gamme d'environ 0.16 mg/kg à environ 13 mg/kg de poids corporel, soit d'environ 8.75 mg à environ 1 g par jour. Dans un mode de réalisation encore préféré, le dosage de l'anticorps est dans la gamme d'environ 0.16 mg/kg à environ 6.5 mg/kg de poids corporel, soit d'environ 8.75 mg à environ 520 mg. Dans un mode de réalisation encore préféré, le dosage de l'anticorps est dans la gamme d'environ 0.16 mg/kg à environ 3.2 mg/kg de poids corporel, soit d'environ 8.75 mg à environ 256 mg. Dans un mode de réalisation encore préféré, le dosage de l'anticorps est dans la gamme d'environ 0.16 mg/kg à environ 1.3 mg/kg de poids corporel, soit d'environ 8.75 mg à environ 104 mg. Dans un mode de réalisation encore préféré, le dosage de l'anticorps est dans la gamme d'environ 0.16 mg/kg à environ 0,64 mg/kg de poids corporel, soit d'environ 8.75 mg à environ 51 mg.

De manière générale, des doses avantageuses sont des doses de 8 à 200 mg par jour, de préférence de 8 à 35 ou de 15 à 70 mg par jour.

Dans un mode de réalisation préféré, le dosage de l'acide caprylique (ou sel de caprylate) est dans la gamme allant de 0,01 mg / kg à environ 500 mg / kg, par exemple 0,1 mg / kg à 300mg / kg, soit d'environ 0.1mg à 20 g par jour.

Le patient peut recevoir par exemple une dose quotidienne d'anticorps anti-TNFα, (de préférence de l'adalimumab) comprise par exemple entre 10 mg et 500mg, de préférence de 8 à 200 mg par jour, de préférence de 8 à 35 ou de 15 à 70 mg par jour

La dose administrée d'acide caprylique (ou sel de caprylate) peut être de préférence comprise entre 100 mg et 4g, de préférence 200 mg et 2g, de préférence entre 300 mg et 1,5g, de préférence entre 750 mg et 1250 mg, et plus préférentiellement entre 900 mg et 1100 mg. Il peut s'agir d'une dose quotidienne ou d'une prise une ou plusieurs fois par semaine.

Dans un mode de réalisation particulier, on peut utiliser l'anticorps et l'acide caprylique (ou sel de caprylate) dans un rapport massique acide caprylique/anticorps au moins égal à 1/1, de préférence 5/1, de préférence 10/1, de préférence 15/1, de préférence 20/1.

Lorsque les deux agents sont administrés de façon séquentielle, la dose est administrée soit le même jour, par au moins deux administrations distinctes, ou alors l'un des agents est administré le premier jour et le second le jour suivant, ou le jour d'après par exemple.

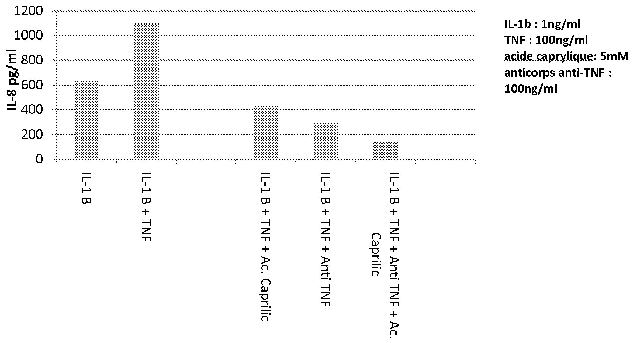

L'exemple suivant et la figure annexée illustrent l'invention sans en limiter la portée.

**Légende de la Figure :**

La figure jointe est un histogramme montrant les taux de sécrétion d'interleukine 8 par des cellules Caco-2, soumises à différentes conditions. La sécrétion est la plus fortement inhibée en présence d'une combinaison acide caprylique + anticorps anti-TNFalpha.

**Exemple : Effet anti-inflammatoire sur lignée Caco-2**

Matériels et méthodes :

Les cellules Caco-2, une lignée cellulaire de cancer du colon, représentent un modèle cellulaire établi, de l'épithelium intestinal humain (Pinto et al, 1983, Biol. Cell, 47, 323-330). Ces cellules sécrètent de l' interleukine 8 (IL-8), qui est une des interleukines majeures dans la pathogenèse des maladies inflammatoires de l'intestin), après activation par l'interleukine 1 beta (IL-Ibeta).

Les inventeurs ont utilisé la lignée cellulaire Caco-2 comme modèle des cellules épithéliales, et ont évalué l'effet de compositions selon l'invention, sur la sécrétion d'interleukine 8 (IL-8), par ces cellules après activation par l'interleukine 1 beta (IL-Ibeta).

Pour cela, les cellules Caco-2, déposées dans des plaques à 24 puits, ont été cultivées pendant 14 jours jusqu'à ce que les cellules soient confluentes et dans un état stable de différenciation. A J14, les cellules ont été précultivées en présence d'acide caprylique, d'anticorps anti-TNFalpha (adalimumab) produit par voie transgénique, et d'une combinaison des deux. 24h plus tard, les cellules ont été stimulées avec 1 ng/ml IL-Ibeta pendant 12 h. Les taux d'IL-8 présents dans les surnageants de culture ont ensuite été mesurés par un dosage ELISA.

Résultats:

L'ajout d'anticorps anti-TNF induit une diminution des taux d'IL-8, et ainsi un potentiel anti-inflammatoire. La combinaison acide caprylique + anticorps anti-TNF apparaît plus fortement inhibitrice de la sécrétion d'IL-8 (cf Figure), montrant un effet plus fortement anti-inflammatoire.

L'utilisation des cellules Caco-2 différenciées après 14 jours de culture, et l'addition d'IL-1 beta, ont permis de reproduire in vitro des conditions similaires à l'inflammation de l'intestin in vivo. Les résultats observés montrent qu'une composition selon l'invention présente un bénéfice thérapeutique potentiel in vivo.

-

**Revendications**

1.  Anticorps anti-TNFa pour une utilisation dans le traitement d'une maladie inflammatoire de l'intestin par administration par voie orale, en combinaison avec une administration concomitante, par voie orale, d'acide caprylique ou d'un sel de caprylate; l'anticorps étant sous la forme d'une composition pharmaceutique adaptée à une libération de l'anticorps ciblée au niveau de l'intestin.

2.  Anticorps anti-TNF$\alpha$, pour une utilisation selon la revendication 1, dans laquelle l'anticorps anti-TNF$\alpha$, est l'adalimumab ou possède la séquence protéique de l'adalimumab.

3.  Anticorps anti-TNF$\alpha$, pour une utilisation selon l'une quelconque des revendications 1 ou 2, où ledit anticorps est un anticorps monoclonal produit dans le lait d'un animal non-humain transgénique.

4.  Anticorps anti-TNF$\alpha$, pour une utilisation selon la revendication 3, où l'animal non-humain transgénique est une chèvre.

5.  Anticorps anti-TNF$\alpha$, pour une utilisation selon l'une des revendications 1 à 4, par administration orale à une dose quotidienne de 8 à 200 mg, de préférence de 8 à 35 ou de 15 à 70 mg d'anticorps anti-TNFa.

6.  Anticorps anti-TNF$\alpha$, pour une utilisation selon l'une des revendications 1 à 5, où la maladie inflammatoire est la maladie de Crohn ou la colite ulcéreuse.

7.  Anticorps anti-TNFa, pour une utilisation selon l'une des revendications 1 à 6, l'anticorps et l'acide caprylique ou le sel de caprylate étant combinés au sein de la même composition pharmaceutique.

8.  Kit comprenant, au sein d'un même emballage :

    - Un premier récipient contenant une composition pharmaceutique pour administration orale, comprenant un anticorps anti-TNFa, qui est de préférence un anticorps monoclonal produit dans le lait d'un animal non-humain transgénique, de préférence une chèvre ; ladite composition pharmaceutique étant adaptée à une libération de l'anticorps ciblée au niveau de l'intestin ;
    - Un deuxième récipient contenant une composition pharmaceutique pour administration orale, comprenant de l'acide caprylique ou un sel caprylate.

**Patentansprüche**

1.  Anti-TNFa-Antikörper zur Verwendung in der Behandlung einer Entzündungskrankheit des Darms mittels oraler Verabreichung in Kombination mit einer gleichzeitigen oralen Verabreichung von Caprylsäure oder einem Salz der Caprylsäure; wobei der Antikörper in Form einer pharmazeutischen Zusammensetzung vorliegt, die für eine gezielte Freisetzung des Antikörpers im Darm geeignet ist.

**2.** Anti-TNF$\alpha$,-Antikörper zur Verwendung gemäß Anspruch 1, wobei der Anti-TNF$\alpha$-Antikörper Adalimumab ist oder die Proteinsequenz von Adalimumab besitzt.

**3.** Anti-TNF$\alpha$,-Antikörper zur Verwendung gemäß einem der Ansprüche 1 oder 2, wo besagter Antikörper ein monoklonaler Antikörper ist, der in der Milch eines transgenen nichthumanen Tiers produziert wird.

**4.** Anti-TNFa-Antikörper zur Verwendung gemäß Anspruch 3, wo das transgene nichthumane Tier eine Ziege ist.

**5.** Anti-TNF$\alpha$,-Antikörper zur Verwendung gemäß einem der Ansprüche 1 oder 4, mittels oraler Verabreichung mit einer Tagesdosis von 8 bis 200 mg, vorzugsweise von 8 bis 35 mg oder von 15 bis 70 mg Anti-TNF$\alpha$,-Antikörper.

**6.** Anti-TNF$\alpha$,-Antikörper zur Verwendung gemäß einem der Ansprüche 1 oder 5, wo die Entzündungskrankheit Morbus Crohn oder Colitis ulcerosa ist.

**7.** Anti-TNF$\alpha$,-Antikörper zur Verwendung gemäß einem der Ansprüche 1 oder 6, wobei der Antikörper und die Caprylsäure oder das Salz der Caprylsäure in derselben pharmazeutischen Zusammensetzung kombiniert sind.

**8.** Kit umfassend in derselben Packung:

- einen ersten Behälter enthaltend eine pharmazeutische Zusammensetzung für eine orale Verabreichung, umfassend einen Anti-TNF$\alpha$,-Antikörper, der vorzugsweise ein monoklonaler Antikörper ist, der in der Milch eines transgenen nichthumanen Tiers, vorzugsweise einer Ziege produziert wird, wobei besagte pharmazeutische Zusammensetzung für eine gezielte Freisetzung des Antikörpers im Darm geeignet ist;
- einen zweiten Behälter enthaltend eine pharmazeutische Zusammensetzung für eine orale Verabreichung, umfassend Caprylsäure oder ein Salz der Caprylsäure.

**Claims**

**1.** An anti-TNFa antibody for use in the treatment of an inflammatory bowel disease by oral administration, in combination with a concomitant administration, by oral administration, of caprylic acid or caprylate salt; wherein the antibody is in the form of a pharmaceutical composition suitable for targeted release of the antibody in the intestine.

**2.** The anti-TNFa antibody for use according to claim 1, wherein the anti-TNFa antibody is adalimumab or has the protein sequence of adalimumab.

**3.** The anti-TNFa antibody for use according to any of claims 1 or 2, wherein said antibody is a monoclonal antibody produced in the milk of a transgenic non-human animal.

**4.** The anti-TNFa antibody for use according to claim 3, wherein the transgenic non-human animal is a goat.

**5.** The anti-TNFa antibody for use according to any of claims 1 to 4, by oral administration of anti-TNFa antibody at a daily dose of 8 mg to 200mg, preferably 8 mg to 35 mg or 15 mg to 70 mg.

**6.** The anti-TNFa antibody for use according to any of claims 1 to 5, wherein the inflammatory disease is Crohn's disease or ulcerative colitis.

**7.** The anti-TNFa antibody, for use according to any of claims 1 to 6, wherein the antibody and caprylic acid or the caprylate salt are combined within the same pharmaceutical composition.

**8.** A kit comprising, within the same package :

- A first container containing a pharmaceutical composition for oral administration, comprising an anti-TNFa antibody, which is preferably a monoclonal antibody produced in the milk of a transgenic non-human animal, preferably a goat; wherein said pharmaceutical composition is suitable for targeted release of the antibody in the intestine;
- A second container containing a pharmaceutical composition for oral administration, comprising caprylic acid or a caprylate salt.

IL-1b : 1ng/ml
TNF : 100ng/ml
acide caprylique: 5mM
anticorps anti-TNF :
100ng/ml

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0264166 A

**Littérature non-brevet citée dans la description**

- **JAREQUI-AMEZAGA et al.** *Journal of Crohn's and colitis,* 2013, vol. 7 (3), 208-212
- **ABREU et al.** *Journal of Crohn's and colitis,* 2013, vol. 7 (2), 175-182
- **HUANG et al.** *Journal of pediatric gastroentology and nutrition,* 2013, vol. 56 (4), e23-6
- **AYALA M ; GAVILONDO J ; RODRÍGUEZ M ; FUENTES A ; ENRÍQUEZ G ; PÉREZ L ; CREMATA J ; PUJOL M.** Production of plantibodies in Nicotiana plants. *Methods Mol Biol.,* 2009, vol. 483, 103-34
- **POLLOCK, D.P. ; J.P. KUTZKO ; E. BIRCK-WILSON ; J.L. WILLIAMS ; Y. ECHELARD ; H.M. MEADE.** Transgenic milk as a method for the production of recombinant antibodies. *Journal of Immunological Methods,* 1999, vol. 231, 147-157
- **HORIUCHI et al.** *Rheumatology,* vol. 49, 1215
- **PINTO et al.** *Biol. Cell,* 1983, vol. 47, 323-330

SEQUENCE LISTING

<110>  Laboratoire Français du Fractionnement et des Biotechnologies

<120>  Composition orale d'anticorps anti-TNFalpha

<130>  B1827PC00

<160>  2

<170>  PatentIn version 3.5

<210>  1
<211>  214
<212>  PRT
<213>  artificial sequence

<220>
<223>  Séquence de chaîne légère de l'adalimumab

<400>  1

```
Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Gly Ile Arg Asn Tyr
            20                  25                  30


Leu Ala Trp Tyr Gln Gln Lys Pro Gly Lys Ala Pro Lys Leu Leu Ile
            35                  40                  45


Tyr Ala Ala Ser Thr Leu Gln Ser Gly Val Pro Ser Arg Phe Ser Gly
        50                  55                  60


Ser Gly Ser Gly Thr Asp Phe Thr Leu Thr Ile Ser Ser Leu Gln Pro
65                  70                  75                  80


Glu Asp Val Ala Thr Tyr Tyr Cys Gln Arg Tyr Asn Arg Ala Pro Tyr
                85                  90                  95


Thr Phe Gly Gln Gly Thr Lys Val Glu Ile Lys Arg Thr Val Ala Ala
            100                 105                 110


Pro Ser Val Phe Ile Phe Pro Pro Ser Asp Glu Gln Leu Lys Ser Gly
            115                 120                 125


Thr Ala Ser Val Val Cys Leu Leu Asn Asn Phe Tyr Pro Arg Glu Ala
            130                 135                 140


Lys Val Gln Trp Lys Val Asp Asn Ala Leu Gln Ser Gly Asn Ser Gln
145                 150                 155                 160


Glu Ser Val Thr Glu Gln Asp Ser Lys Asp Ser Thr Tyr Ser Leu Ser
```

                    165                 170                     175


Ser Thr Leu Thr Leu Ser Lys Ala Asp Tyr Glu Lys His Lys Val Tyr
            180                 185                 190


Ala Cys Glu Val Thr His Gln Gly Leu Ser Ser Pro Val Thr Lys Ser
            195                 200                 205


Phe Asn Arg Gly Glu Cys
            210


<210>   2
<211>   224
<212>   PRT
<213>   artificial sequence

<220>
<223>   séquence de chaîne lourde de l'adalimumab

<400>   2

Glu Val Gln Leu Val Glu Ser Gly Gly Gly Leu Val Gln Pro Gly Arg
1               5                   10                  15


Ser Leu Arg Leu Ser Cys Ala Ala Ser Gly Phe Thr Phe Asp Asp Tyr
            20                  25                  30


Ala Met His Trp Val Arg Gln Ala Pro Gly Lys Gly Leu Glu Trp Val
            35                  40                  45


Ser Ala Ile Thr Trp Asn Ser Gly His Ile Asp Tyr Ala Asp Ser Val
            50                  55                  60


Glu Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
            85                  90                  95


Ala Lys Val Ser Tyr Leu Ser Thr Ala Ser Ser Leu Asp Tyr Trp Gly
            100                 105                 110


Gln Gly Thr Leu Val Thr Val Ser Ser Ala Ser Thr Lys Gly Pro Ser
            115                 120                 125


Val Phe Pro Leu Ala Pro Ser Ser Lys Ser Thr Ser Gly Gly Thr Ala
            130                 135                 140


Ala Leu Gly Cys Leu Val Lys Asp Tyr Phe Pro Glu Pro Val Thr Val
145                 150                 155                 160

```
Ser Trp Asn Ser Gly Ala Leu Thr Ser Gly Val His Thr Phe Pro Ala
            165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
            195                 200                 205

Lys Pro Ser Asn Thr Lys Val Asp Lys Lys Val Glu Pro Lys Ser Cys
    210                 215                 220
```